# EUROPÄISCHE PATENTSCHRIFT

(11) **EP 1 719 535 B1**
(45) Veröffentlichungstag und Bekanntmachung des Hinweises auf die Patenterteilung: **18.12.2013**
(21) Anmeldenummer: 06008160.1
(22) Anmeldetag: 20.04.2006
(51) Int. Cl.: A61M 16/04

(54) **TRACHEOSTOMAPLATZHALTER**
TRACHEOSTOMA PLACEHOLDER
DISPOSITIF D'ACCES POUR TRACHEOTOMIE

(30) Priorität: 02.05.2005 DE 102005020725
(43) Veröffentlichungstag der Anmeldung: 08.11.2006
(73) Patentinhaber: ISKIA GmbH & Co. KG, 38829 Harsleben (DE)
(72) Erfinder: Neubauer, Norbert, 38820 Halberstadt (DE); Maltzahn, Meik, 38820 Halberstadt (DE); Freigang, Bernd, Prof. Dr.-med., 39110 Magdeburg (DE)
(74) Vertreter: Pfeiffer, Rolf-Gerd

(56) Entgegenhaltungen:
- WO-A-86/06970
- DE-A1- 19 636 050
- DE-B3-102005 020 725
- US-A- 3 137 299
- US-A- 3 459 175
- US-A- 4 269 184
- US-A- 5 184 610
- US-A- 5 683 458

## Beschreibung

Die Erfindung betrifft einen Tracheostomaplatzhalter zum Abdichten einer vorübergehenden Tracheaeröffnung und Bildung einer sicheren Zugangsmöglichkeit zur Luftröhre, der bevorzugt in folgenden Anwendungsgebieten Einsatz findet:
- Entwöhnung von Luftröhrenkanülen und Beatmungstuben nach Langzeitbeatmung oder nach einer Kehlkopf- oder Luftröhrenchirurgie,
- Gewährleistung eines sicheren Zugangs zur Luftröhre bei Gefahr einer Atemwegsverlegung oder -blutung nach chirurgischen Eingriffen an Pharynx/Larynx, Tumoren,
- Sekretabsaugmöglichkeit aus tieferen Atemwegen bei schweren pulmonalen oder neurologischen Erkrankungen,
- bei wiederkehrenden Atemwegsverlegungen im Bereich des Schlundes oder des Kehlkopfes, wie im Falle von obstruktiver Schlafapnoe, gestörter Glottismotorik oder bei bestrahlungsbedingter Verschwellung.
Ebenso kann die erfindungsgemäße Lösung in Sonderanwendungen, wie bei Einatemhindernissen, z.B. bei doppelseitiger Stimmbandlähmung, Einsatz finden.

Es sind verschiedene Tracheostomaplatzhalter bekannt. So bspw. ein sogenannter "Barton-MayoTracheostoma Button", der aus einem einstückigen Teil bestehend aus einer Ventilaufnahme, einem Tracheostomaschenkel und einer in das Stoma eindrückbaren knopfartigen, konzentrischen Abschluss besteht. Dieser Platzhalter hat den Nachteil, dass er den jeweiligen anatomischen Besonderheiten nur durch eine spezielle Neuanfertigung anpassbar wäre, über keine besonders gute Dichtwirkung verfügt und vor allem beim Patienten durch eine relativ scharfkantige Anlage an der Halshaut zu Schwellungen und Schmerzen führt. Bei anderen bekannten Abstandshaltern (z.B. US-PS 4,269,184) wird versucht, die trachealseitige Anlage des Abstandshalters dadurch zu verbessern, dass an dem Tracheostomaschenkel diametral gegenüberstehend zwei gleichlange, lappenartige Anlagen vorgesehen sind. Auch diese Lappen stellen keine sichere Anlage an die Trachea dar und neigen sehr leicht zur Dislokation. Allen bekannten Tracheostomaplatzhaltern zum Abdichten einer vorübergehenden Tracheaeröffnung und Bildung einer sicheren Zugangsmöglichkeit zur Luftröhre ist gemein, dass die Trageeigenschaften beim Patienten als unangenehm empfunden werden.

Weiterhin sind für andere Einsatzgebiete, nämlich für die Ventilabdichtung des Luftröhrenstumpfes nach einer Kehlkopftotalentfernung, Tracheostoma-Verschlüsse aus DE-U-85 14 859 und US-PS 5,683,458 bekannt. Eine Zuführmöglichkeit bspw. einer Atemkanüle oder eines Beatmungstubus ist bei diesen Lösungen jedoch nicht möglich.

Schließlich ist aus DE 196 36 050 C2 ein Tracheostomaplatzhalter bekannt, mit dem versucht wird, vorstehend genannte Probleme von Tracheostomaplatzhaltern zu beheben. Dieser besteht aus einem rohrförmigen Tracheostomaschenkel, der einseitig in einen einstückig angeformten und in einer ersten Dimension gewölbt ausgebildeten Trachealschenkel ausläuft, wobei der Trachealschenkel in einer zur ersten Dimension senkrecht verlaufenden Dimension amittig zur Tracheostomaschenkelachse derart angebunden ist, dass er unterschiedlich lange Überhangbereiche aufweist, wobei der Tracheostomaschenkel an dem, dem Trachealschenkel abseitigen Ende mit einem Verschlussmittel versehbar ist, wobei die Überhangbereiche die mit mehreren ausgedünnten Trachealschenkelabschnitten nach der dortigen Lehre zu versehen sind, und dem Tracheostomaschenkel unterbrochene Rastmittel zugeordnet sind, die die Aufnahme und Fixierung eines Schildes in einem variabel anpassbaren Abstand zur Anlagefläche des Trachealschenkels an die Trachea gewährleisten sollen. Letzteres ist durch die vorgesehenen, in einem festen Abstand anbringbare Rastmittel nur bedingt möglich. Außerdem wird auch diese Konstruktion vom Patienten im Tragekomfort als unangenehm empfunden und führt infolge der dort vorgesehenen Art und Weise der kantigen Rastmittelausbildung sogar zu Entzündungen im Tracheostoma. Im übrigen sind derartige Verrippungen aus sogenannten T-Tuben für einen vergleichbaren Verwendungszwecks schon seit Jahrzehnten bekannt, so dass das Wesen vorstehender zitierter Erfindung in den unterschiedlich lang ausgebildeten und mit Ausdünnungen versehenen Überhangbereichen bestehen dürfte.

Vorliegender Erfindung liegt daher die Aufgabe zugrunde, einen Tracheostomaplatzhalter zum Abdichten einer vorübergehenden Tracheaeröffnung und Bildung einer sicheren Zugangsmöglichkeit zur Luftröhre anzugeben, der die Nachteile des Standes der Technik behebt, einen sicheren und weitestgehend selbstdichtenden Sitz in der Luftröhrenöffnung gewährleistet und eine zusätzliche Tracheostomaabdichtung gewährleistet.

Die Aufgabe wird durch die Merkmale des Patentanspruchs 1 gelöst. Vorteilhafte weitere Ausbildungen sind durch die nachgeordneten Ansprüche erfasst.

Die Erfindung soll nachstehend anhand eines Ausführungsbeispiels und schematischer Zeichnungen näher erläutert werden. Es zeigen:
- Fig. 1: eine Ausbildung eines Tracheostomaplatzhalters mit den erfindungswesentlichen Bestandteilen in perspektivischer Ansicht,
- Fig. 2: links die Ausbildung eines Dichtungskegels nach Figur 1 in geschnittener Seitenansicht und rechts in Draufsicht,
- Fig. 3: die Ausbildung eines Tracheostomaschenkels mit sich anschließenden Trachealschenkeln in seitlicher Ansicht und rechts daneben im dazu senkrechten seitlichen Schnitt und
- Fig. 4: in einer Detaildarstellung in geschnittener Draufsicht die Lagen, die die Trachealschenkel einnehmen können.

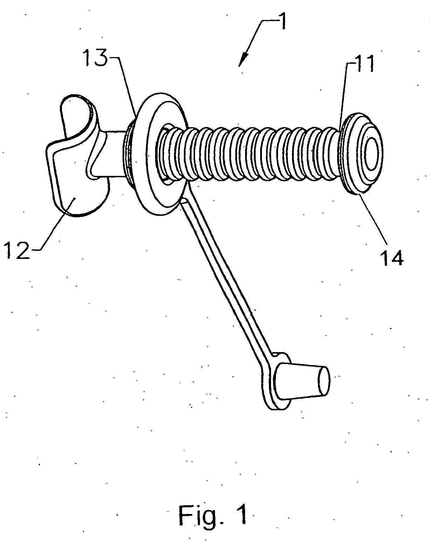

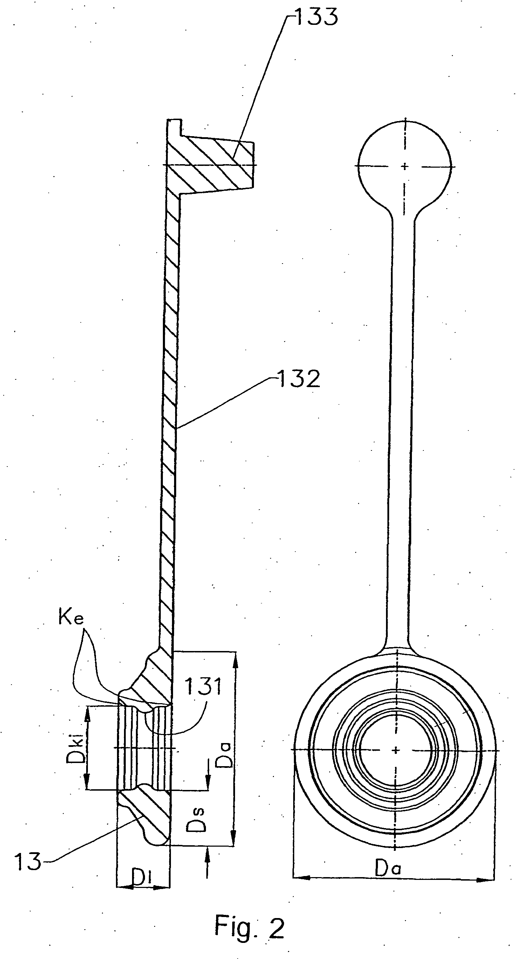

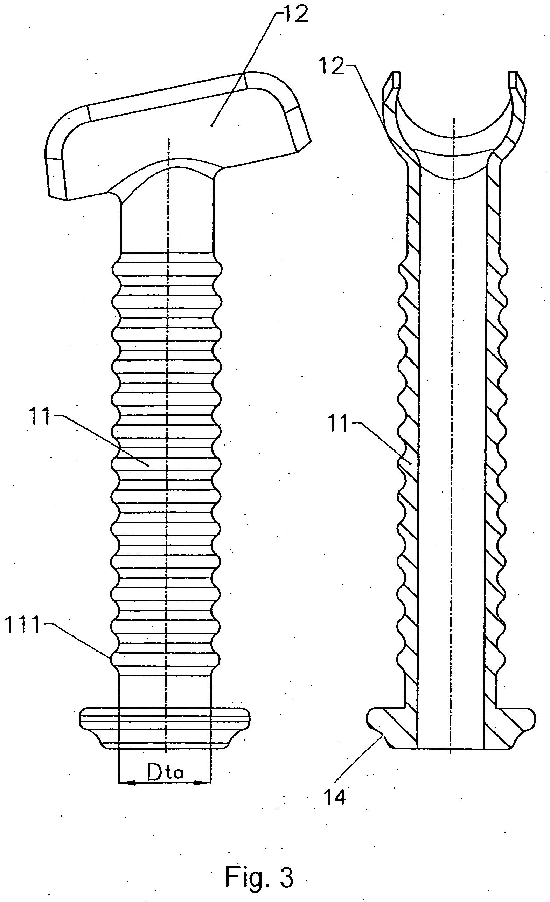

In Figur 1 ist ein Tracheostomaplatzhalter **1** nach vorliegender Erfindung in perspektivischer Ansicht dargestellt, der aus einem Tracheostomaschenkel **11** und Trachealschenkeln **12** besteht; beide Teile bilden bevorzugt eine einstückige Einheit. Im Gegensatz zum bekannten Stand der Technik weist der Tracheostomaschenkel **11** im Wesentlichen eine zylindrische Oberfläche, frei von unstetigen Profilierungen, auf. Auf diesen Tracheostomaschenkel **11** ist ein den Schenkel umfassender auf- und verschiebbarer und in Tracheostomarichtung verjüngt zulaufender elastischer Dichtungskegel **13** vorgesehen ist, dessen lichter, die Anlage am Tracheostomaschenkel **11** bewirkender Innendurchmesser **Dki** (vgl. Fig. 2) in der Größenordnung von 5% geringer festgelegt ist als der in Anlage zum Dichtungskegel effektiv wirkende Außendurchmesser **Dta** (vgl. Fig. 3) des Tracheostomaschenkels **11.** Aus Figur 1 ist weiterhin ersichtlich, dass unmittelbar am Dichtungskegel **13** ein mit einem Stopfen **133** versehener Strang **132** vorgesehen ist, wobei die Teile **13, 132** und **133** vorzugsweise als einstückige, gespritzte Einheit gefertigt sind. Weiterhin ist im Rahmen vorliegender Erfindung vorgesehen, das patientenabseitige Ende des Tracheostomaplatzhalters 1 mit einem Sicherungsmittel zu versehen. Dieses Sicherungsmittel verhindert, dass der Dichtungskegel **13,** insbesondere bei zylindrisch glatter Ausbildung des Tracheostomaschenkels **11** beim Einsetzen in das Stoma vor der endgültigen Platzierung versehentlich abgezogen werden kann. Im Rahmen der Erfindung ist es besonders vorteilhaft dieses Sicherungsmittel als Sicherungsring **14** auszubilden, der in einem Arbeitsgang an den Tracheostomaschenkel **11** mit angespritzt wird und somit ebenfalls ein einstückiges Element des Tracheostomaplatzhalters ist. Beim Einsetzen in das Stoma ist mit Hilfe dieses Sicherungsrings **14** ein unkontrolliertes Verrutschen des Tracheostomaplatzhalters in das Stoma bei versehentlicher Entfernung des Dichtungskegels **13** ausgeschlossen und bei schlechter Platzierung kann der Tracheostomaplatzhalter **1** damit wieder leicht entnommen werden. Nachdem der Tracheostomaplatzhalter **1** exakt positioniert ist, besteht keine weitere Notwendigkeit zur zusätzlichen Sicherung und der Sicherungsring **14** kann einfach abgeschnitten werden. Die im Beispiel dargestellte Ausbildung genannten Sicherungsmittels beschränkt die Erfindung nicht auf die Ausbildung als einstückigem Sicherungsring **14.** Auch andersartig ausgebildete Formen, die den gleichen Zweck erfüllen, sollen unter die Erfindung fallen.

Die spezielle Ausbildung des Dichtungskegels **13** ist in Figur 2 im Detail dargestellt. Im linken Teil der Abbildung ist der Dichtungskegel **13** mit den ihm zugeordneten Baugruppen **132** und **133** im seitlichen Schnitt dargestellt. Es ist erkenntlich, dass der Dichtungskegel selbst aus einem Vollmaterial besteht, wobei der Dichtungskegel **13** im Außendurchmesser **Da** wenigstens doppelt so groß bemessen ist, im Beispiel 30 mm, wie der Außendurchmesser **Dta** des Tracheostomaschenkels **11,** im Beispiel 11 mm, wobei die laterale Ausdehnung **DI** der Kegelanlagefläche im wesentlichen gleichlang zur senkrecht dazu verlaufenden Ausdehnung **Ds** der Kegelbasisfläche festgelegt ist. Durch die Maßgabe, dass der Innendurchmesser **Dki,** im Beispiel 9,5 mm, des Dichtungskegels in der Größenordnung von 5% geringer festgelegt ist als der effektiv wirkende Außendurchmesser **Dta,** im Beispiel 11 mm, des Tracheostomaschenkels **11,** wird bei Einsatz von Silikon für den Dichtungskegel und den Tracheostomaschenkel eine hinreichend hohe Haftreibung erzielt, um dem Dichtungskegel eine hinreichende Lagestabilisierung bei gleichzeitiger Verschiebbarkeit zu verleihen. Im Gegensatz zum bekannten Stand der Technik sind für den Dichtungskegel keine zusätzlichen, gesondert anzubringenden Haltebänder erforderlich, was den Tragekomfort für den Patienten zusätzlich erhöht, diese Möglichkeit aber nicht ausschließt. In Fig. 2 ist weiterhin ersichtlich, dass der Dichtungskegel **13,** insbesondere an den Enden **Ke** seiner Anlageflächen zum Tracheostomaschenkel **11** verrundet ausgebildet ist, was zum einen das Aufsetzen und Verschieben des Dichtungskegels auf den Tracheostomaschenkel erleichtert zum anderen aber auch eine angenehme zusätzliche Dichtungsfläche am Tracheostoma gewährleistet.

Um jedoch eine weitere Erhöhung eines gesicherten Sitzes des Dichtungskegels **13** auf dem Tracheostomaschenkel **11** zu erreichen, liegt es im Rahmen der Erfindung, den Tracheostomaschenkel **11** in seiner Längserstreckungsrichtung mit einer stetig wellenförmig verlaufenden und in sich umfangsmäßig geschlossenen Oberflächenprofilierung **111** zu versehen (vgl. Fig. 3), welche eine weitere Modifikation des Dichtungskegels **13,** wie weiter unter beschrieben, nach sich zieht. Im Beispiel wurden beste Ergebnisse erzielt mit einer Profilierung, die eine Welligkeit der Oberflächenprofilierung in Tracheostomaschenkellängserstreckungsrichtung mit einem annähernd sinusförmigen Verlauf mit einer Periodenlänge in der Größenordnung von 3 mm aufweist.

Um dem Dichtungskegel **13** auf einem nach Fig. 2 ausgebildeten Tracheostomaschenkel **11** den erforderliche Halt und festen Sitz zu geben, ist der Dichtungskegel **13** innenseitig mit einer Wulst **131** versehen, die bezüglich ihres Oberflächenprofils korrespondierend zu der Oberflächenprofilierung **111** des Tracheostomaschenkels **11** komplementär angepasst ausgebildet ist. Das heißt, die Wulst soll sich optimal in ein Wellental der Oberflächenprofilierung **111** einpassen. Analog liegt es ebenso im Rahmen der Erfindung, statt genannter Wulst eine oder zwei im Dichtungskegel umlaufende Nuten (nicht dargestellt) vorzusehen, die dann die Aufnahme eines Wellenbergs der Oberflächenprofilierung **111** gewährleisten. Wesentlich bei beiden Ausführungen ist, dass ebenfalls im Gegensatz zum Stand der Technik sowohl die Oberflächenprofilierungen **111** auf dem Tracheostomaschenkel **11** als auch die Wulst **131** (respektive Nut) umlaufend in sich geschlossen sind und keine Durchbrüche aufweisen, was im Sinne des gesicherten Sitzes als auch der Reinigung Vorteile bietet. Die Wulst oder Nut mittig im Dichtungskegel anzubringen ist besonders vorteilhaft, beschränkt die Erfindung jedoch nicht einzig auf diese Art der Anbringung. Jedoch sollten die Wulst oder Nut vornehmlich in Richtung des verjüngten Dichtungskegelteils angeordnet sein.

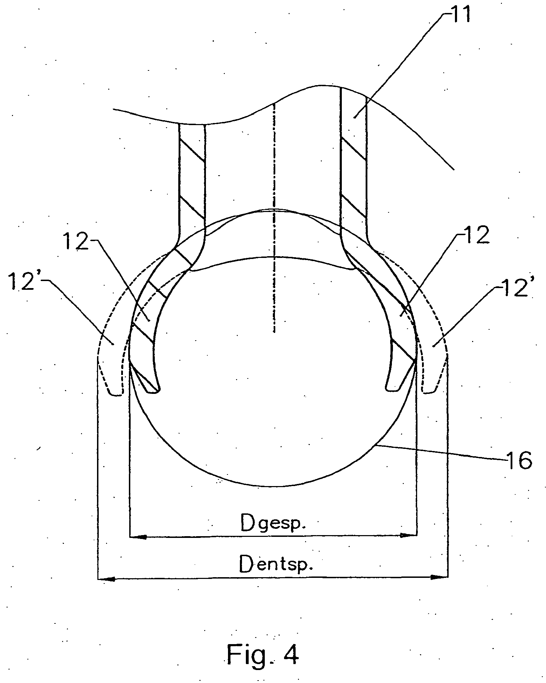

Eine weitere wesentliche Verbesserung des Tracheostomaplatzhalters **1** wird im Gegensatz zu bekannten Platzhaltern dadurch erreicht, dass den Trachealschenkeln **12** zumindest in ihrer Außenberandung, wobei die geschlossene Kreislinie **16** in Figur 4 symbolisch für die Luftröhre steht, eine von einer Kreisform sich vergrößernde abweichende Geometrie im entspannten Zustand gegeben ist. Das heißt, dass die Trachealschenkel **12** im Schnitt senkrecht zur Einlage in der Luftröhre so ausgebildet sind, dass sie in Abweichung von der Anlage an eine Kreisform **16,** die die Lage der Trachealschenkel **12** im in die Luftröhre eingelegten, gespannten Zustand beschreibt, eine gespreizte Ausgangslage **12'** im ungespannten Zustand einnehmen. Dadurch üben die Trachealschenkel **12** eine gewisse Vorspannkraft aus, die ihre gesicherte Lage und Dichtung in der Luftröhre bewirken.
Im Beispiel sind bei einer durchschnittlichen Materialdicke der Trachealschenkel **12** in der Größenordnung von 1,5 mm die Schenkel so ausgeführt, dass der Öffnungsdurchmesser **Dentsp.** der Trachealschenkel **12** im entspannten Zustand bis zu 20% größer ist als der Öffnungsdurchmesser **Dgesp.** der Trachelaschenkel **12** im gespannten Zustand bei Einlage in die Luftröhre. Bei genannten Wandstärken und Verwendung von Silikon als Material für die Trachealschenkel wird damit ein ausreichender und sich an die meisten anatomischen Besonderheiten der Patienten angepasster Anpressdruck in der Luftröhre realisiert, ohne vom Patienten als Druck empfunden zu werden.
Ebenfalls im Gegensatz zu bekannten Realisierungsformen nach dem Stand der Technik müssen hier nicht unterschiedlich lange Tracheostomaschenkel angeboten werden, sondern durch die direkte Anbringung des Stopfens **133** an den Dichtungskegel **13** ist der Tracheostomaschenkel **11** individuell an den Patienten angepasst kürzbar und der Stopfen **133** sorgt in nächster Nähe am Dichtungskegel **13** für den Verschluss des nach außen offenen Tracheostomaschenkelendes.

Mit dem geschaffenen Tracheostomaplatzhalter liegt somit eine Vorrichtung für multivalente Einsatzgebiete vor, die den anatomischen Besonderheiten des Patienten Rechnung trägt und einen sicheren und komfortablen Sitz gewährleistet. Durch die beschriebene Art der Ausbildung des Dichtungskegels **13** bewirkt dieser im aufgeschobenen Zustand eine zusätzliche äußere Tracheostomaabdichtung und sein verrundet ausgeführtes Ende als auch die stetigen Verrundungen der beschriebenen Oberflächenprofilierung **111,** sollten sie selbst teilweise ins Tracheostoma hineinragen, bewirken keine Entzündungen im Stoma.

Alle in der Beschreibung, dem Ausführungsbeispiel und den nachfolgenden Zeichnungen erkennbaren Merkmale können sowohl einzeln als auch in beliebiger Kombination miteinander erfindungswesentlich sein.

### Bezugszeichenliste

- 1 -: Tracheostomaplatzhalter
- 11 -: Tracheostomaschenkel
- 12 -: Trachealschenkel
- 12' -: Trachealschenkel in entspannter Ausgangslage
- 13 -: Dichtungskegel
- 131 -: Wulst
- 132 -: Strang
- 133 -: Stopfen
- 14 -: Sicherungsmittel/Sicherungsring
- 16 -: kreisförmige Luftröhre
- Dgesp. -: Öffnungsdurchmesser der Trachealschenkel im gespannten Zustand
- Dentsp. -: Öffnungsdurchmesser der Trachealschenkel Im entspannten Zustand
- Da -: Außendurchmesser des Dichtungskegels
- Dki -: Innendurchmesser des Dichtungskegels
- DI -: laterale Ausdehnung der Kegelanlagefläche
- Ds -: Ausdehnung der Dichtungskegelbasisfläche
- Dta -: Außendurchmesser des Tracheostomaschenkels 11
- Ke -: Verrundungen der Dichtungskegelenden

## Patentansprüche

1. Tracheostomaplatzhalter (**1**) zum Abdichten einer vorübergehenden Tracheaeröffnung und Bildung einer sicheren Zugangsmöglichkeit zur Luftröhre, bestehend aus einem rohrförmigen Tracheostomaschenkel (**11**), an den einseitig gewölbt ausgebildete Trachealschenkel (**12**) angeformt sind, **dadurch gekennzeichnet, dass** der Tracheostomaschenkel (**11**) im wesentlichen eine zylindrische Oberfläche, frei von unstetigen Profilierungen, aufweist, auf den umfassend ein auf- und verschiebbarer und in Tracheostomarichtung verjüngt zulaufender elastischer Dichtungskegel (**13**) vorgesehen ist, dessen lichter, die Anlage am Tracheostomaschenkel (**11**) bewirkender Innendurchmesser (**Dki**) in der Größenordnung von 5% geringer festgelegt ist als der in Anlage zum Dichtungskegel effektiv wirkende Außendurchmesser (**Dta**) des Tracheostomaschenkels (**11**), dass den Trachealschenkeln (**12**) zumindest in ihrer Außenberandung (**16**) eine von einer Kreisform sich vergrößernde abweichende Geometrie im entspannten Zustand gegeben ist, und dass der Tracheostomaplatzhalter (**1**) am der Trachealschenkel (**12**) gegenüberliegenden Ende mit einem Sicherungsmittel (**14**) versehen ist.

2. Tracheostomaplatzhalter nach Anspruch 1, **dadurch gekennzeichnet, dass** der Dichtungskegel (**13**) im Außendurchmesser (**Da**) wenigstens doppelt so groß bemessen ist, wie der Außendurchmesser (**Dta**) des Tracheostomaschenkels (**11**), wobei die laterale Ausdehnung (**DI**) der Kegelanlagefläche im wesentlichen gleichlang zur senkrecht dazu verlaufenden Ausdehnung (**Ds**) der Kegelbasisfläche festgelegt ist.

3. Tracheostomaplatzhalter nach Anspruch 1, **dadurch gekennzeichnet, dass** der Dichtungskegel (**13**), insbesondere an den Enden (**Ke**) seiner Anlageflächen zum Tracheostomaschenkel (**11**) verrundet ausgebildet ist.

4. Tracheostomaplatzhalter nach Anspruch 1, **dadurch gekennzeichnet, dass** der Tracheostomaschenkel (**11**) mit einer stetig wellenförmig verlaufenden und in sich umfangsmäßig geschlossenen Oberflächenprofilierung (**111**) versehen ist.

5. Tracheostomaplatzhalter nach Anspruch 4, **dadurch gekennzeichnet, dass** der Welligkeit der Oberflächenprofilierung ein annähernd sinusförmiger Verlauf mit einer Periodenlänge in der Größenordnung von 3 mm gegeben ist.

6. Tracheostomaplatzhalter nach Anspruch 1 und 4, **dadurch gekennzeichnet, dass** der Dichtungskegel (**13**) innenseitig mit einer Wulst (**131**) oder Nut versehen ist, die bezüglich ihres Oberflächenprofils korrespondierend zu der Oberflächenprofilierung (**111**) des Tracheostomaschenkels (**11**) komplementär angepasst ausgebildet ist.

7. Tracheostomaplatzhalter nach Anspruch 5, **dadurch gekennzeichnet, dass** die Wulst (**131**) oder Nut mittig im Dichtungskegel (**13**) angeordnet ist.

8. Tracheostomaplatzhalter nach Anspruch 1, **dadurch gekennzeichnet, dass** die Trachealschenkel (**12**) im Schnitt senkrecht zur Einlage in der Luftröhre so ausgebildet sind, dass sie in Abweichung von der Anlage an eine Kreisform (**16**), die die Lage der Trachealschenkel (**12**) im in die Luftröhre eingelegten, gespannten Zustand beschreibt, eine gespreizte Ausgangslage (**12'**) im ungespannten Zustand einnehmen.

9. Tracheostomaplatzhalter nach Anspruch 7, **dadurch gekennzeichnet, dass** der Öffnungsdurchmesser (**Dentsp.**) der Trachealschenkel (**12**) im entspannten Zustand bis zu 20% größer ist als der Öffnungsdurchmesser (**Dgesp**.) der Trachelaschenkel (**12**) im gespannten Zustand bei Einlage in die Luftröhre.

10. Tracheostomaplatzhalter nach Anspruch 1 und 8, **dadurch gekennzeichnet, dass** die Wandstärken der Trachealschenkel (**12**) und des Tracheostomaschenkels (**11**) etwa gleich dick in einer Größenordnung von 1,5 mm festgelegt sind.

11. Tracheostomaplatzhalter nach Anspruch 1, **dadurch gekennzeichnet, dass** am Dichtungskegel (**13**) ein mit einem Stopfen (**133**) versehener Strang (**132**) vorgesehen ist.

12. Tracheostomaplatzhalter nach Anspruch 10, **dadurch gekennzeichnet, dass** der Dichtungskegel (**13**), der Stopfen (**133**) und der Strang (**132**) als einstückiges Spritzteil gefertigt sind.

13. Tracheostomaplatzhalter nach Anspruch 1, **dadurch gekennzeichnet, dass** der Tracheostomaschenkel (**11**) und die Trachealschenkel (**12**) als einstückiges Spritzteil gefertigt sind.

14. Tracheostomaplatzhalter nach Anspruch 1, **dadurch gekennzeichnet, dass** das Sicherungsmittel durch einen angespritzten und abtrennbaren Sicherungsring (**14**) gebildet ist, der in seinem Außendurchmesser so bemessen ist, dass er ein unbeabsichtigtes Abstreifen des Dichtungskegels (**13**) verhindert.

15. Tracheostomaplatzhalter nach Anspruch 13 und 14, **dadurch gekennzeichnet, dass** der Tracheostomaschenkel (**11**), die Trachealschenkel (**12**) und der Dichtungskegel (**13**) als einstückiges Spritzteil gefertigt sind.

16. Tracheostomaplatzhalter nach einem der vorstehenden Ansprüche, **dadurch gekennzeichnet, dass** sämtliche Teile des Tracheostomaplatzhalters (**1**) aus einem Silikonmaterial gefertigt sind.

## Claims

1. Tracheostoma in-place-holder (1) for sealing a temporary tracheostoma and for providing a secure accessibility to the trachea, comprising a tubular tracheostoma arm (11) to which, on one end, curvedly designed tracheal legs (12) are formed, **characterized in that** the tracheostoma arm (11) has a substantially cylindrical surface, devoid of discontinuous profiles, on said tracheostoma arm (11) an elastic sealing cone (13) is encompassingly provided being slidable onto the former and displaceable and being tapering towards the tracheostoma, the inner diameter (Dki) of said sealing cone (13) which effects the bearing on the tracheostoma arm (11) is selected a 5% lower size than the outer diameter (Dta) of the tracheostoma arm (11), when effectively acting upon the sealing cone when attached to the latter, and **in that** the outside diameter of the tracheal legs (12) in a stress relieved state at least in its outer rim (16) is given one from a circular shape increasingly departing geometry, and **in that** the tracheostoma in-place-holder (1) is provided with a securing means (14) at the end opposing the tracheal legs (12).

2. Tracheostoma in-place-holder as claimed in claim 1, **characterized in that** the outer diameter (Da) of the sealing cone (13) is at least dimensioned double the size of the outer diameter (Dta) of the tracheostoma arm (11), whereby the lateral extension (DI) of the bearing face of the cone is selected substantially of the same length as the extension (Ds) of the base of the cone being at right angles to the former.

3. Tracheostoma in-place-holder as claimed in claim 1, **characterized in that** the sealing cone (13) is roundly designed in particular at the ends (Ke) of its bearing faces attaching the tracheostoma arm (11).

4. Tracheostoma in-place-holder as claimed in claim 1, **characterized in that** the tracheostoma arm (11) is provided with a circumferentially closed continuously wavy surface profile (111).

5. Tracheostoma in-place-holder as claimed in claim 4, **characterized in that** the waviness of the surface profile is substantially sinusoidal in its course with a length of period in an order of size of 3 mm.

6. Tracheostoma in-place-holder as claimed in claim 1 and 4, **characterized in that** the sealing cone (13) is provided with a collar (131) or a groove on its interior side the surface profile of which being designed to complementary match the surface profile (111) of the tracheostoma arm (11).

7. Tracheostoma in-place-holder as claimed in claim 5, **characterized in that** the collar (131) or the groove is arranged centrally within the sealing cone (13).

8. Tracheostoma in-place-holder as claimed in claim 1, **characterized in that** the tracheal legs (12) are, in a section at right angles to their insertion into the trachea, such designed that they will take a spread position (12') in their unstressed state, departing from a circular form (16), when attached, which describes the position of the tracheal legs (12) in the tensioned state when inserted into the trachea.

9. Tracheostoma in-place-holder as claimed in claim 7, **characterized in that** the opening diameter (Dentsp) of the tracheal legs (12) is up to 20% larger in the unstressed state than the opening diameter (Dgesp) of the tracheal legs (12) in their stressed state when inserted into the trachea.

10. Tracheostoma in-place-holder as claimed in claim 1 and 8, **characterized in that** the wall thickness of the tracheal legs (12) and of the tracheostoma arm (11) are selected about equally thick in an order of size of 1,5 mm.

11. Tracheostoma in-place-holder as claimed in claim 1, **characterized in that** the sealing cone (13) has a rod (132) provided with a plug (133).

12. Tracheostoma in-place-holder as claimed in claim 10, **characterized in that** the sealing cone (13), the plug (133) and the rod (132) are made as a single piece by die-casting.

13. Tracheostoma in-place-holder as claimed in claim 1, **characterized in that** the tracheostoma arm (11) and the tracheal legs (12) are made as a single piece by die-casting.

14. Tracheostoma in-place-holder as claimed in claim 1, **characterized in that** a securing means is formed by a jetted-on safety ring (14) being separable, the outer diameter of said safety ring being such dimensioned to prevent an inadvertent stripping off of the sealing cone (13).

15. Tracheostoma in-place-holder as claimed in claim 13 and 14, **characterized in that** the tracheostoma arm (11), the tracheal legs (12) and the sealing cone are made as a one piece part by die-casting.

16. Tracheostoma in-place-holder as claimed in any of the preceding claims, **characterized in that** all parts of the tracheostoma in-place-holder (1) are made of a silicone material.

## Revendications

1. Le support de trachéostome (**1**), ayant pour objectif d'assurer l'étanchéité d'une ouverture temporaire de la trachée et la création d'un accès fiable à la trachée, comprenant une branche de trachéostome (**11**) en forme de tube sur laquelle sont adaptées des branches trachéales bombées sur un côté (**12**), est **caractérisé en ce que** la surface de la branche de trachéostome (**11**) est essentiellement cylindrique, ne présente aucun profilage discontinu et que sur laquelle est montée une cône d'étanchéité souple (**13**) ayant une forme conique en direction du trachéostome, qui par un mouvement glissant peut s'ouvrir ou se fermer et dont le diamètre intérieur (**Dki**) assurant la pression contre la branche du trachéostome (**11**) est de 5% plus petit que le diamètre extérieur (**Dta**) assurant la pression effective contre le cône d'étanchéité de la branche de trachéostome (**11**) de sorte qu'au moins le bord extérieur (**16**) des branches trachiales (**12**) présente, en état détendu, une géométrie grandissante qui diffère de la forme circulaire et que le support du trachéostome (**1**) est dotée d'un dispositif de sécurité (**14**) situé à l'extrémité opposée à la branche trachéale (**12**).

2. Le support de trachéostome selon la revendication 1 est **caractérisé en ce que** le diamètre extérieur (**Da**) du cône d'étanchéité (**13**) est au moins deux fois plus grand que le diamètre extérieur (**Dta**) de la branche de trachéostome(**11**), l'étendue latérale (**DI**) de la surface de contact sur le cône étant dans l'essentiel aussi longue que l'étendue perpendiculaire (**Ds**) de la surface de la base du cône.

3. Le support de trachéostome selon la revendication 1 est **caractérisé en ce que** le cône d'étanchéité (**13**) a une forme arrondie notamment aux extrémités (**Ke**) de ses surfaces de contact avec la branche du trachéostome (**11**).

4. Le support de trachéostome selon la revendication 1 est **caractérisé en ce que** la branche du trachéostome (**11**) est dotée à sa surface d'un profil (**111**) ondulé continu et fermé sur sa circonférence.

5. Le support de trachéostome selon la revendication 4 est **caractérisé en ce que** l'ondulation du profil de surface se distingue par un parcours approximativement sinusoïdal aux ondes périodiques de 3 mm.

6. Le support de trachéostome selon les revendications 1 et 4 est **caractérisée en ce que** le cône d'étanchéité (**13**) est doté sur sa face intérieure d'un bourrelet (**131**) ou d'une rainure dont la surface profilée a une forme adaptée de manière complémentaire à la surface profilée (**111**) de la branche du trachéostome (**11**).

7. Le support de trachéostome selon la revendication 5 est **caractérisé en ce que** le bourrelet (**131**) ou la rainure sont disposés de manière axiale dans le cône d'étanchéité (**13**).

8. Le support de trachéostome selon la revendication 1 est **caractérisé en ce que** les branches trachéales (**12**) vues en coupe perpendiculaire par rapport à la position dans la trachée, sont formées de sorte qu'elles se trouvent, en état non tendu, dans une position initiale déployée ce qui est différent de la situation où il s'agit du contact avec une forme circulaire (**16**) qui décrit la position des branches trachéales (**12**) dans la trachée en état tendu.

9. Le support de trachéostome selon la revendication 7 est **caractérisé en ce que** le diamètre de l'ouverture (**Dentsp**.) des branches trachéales (**12**) en état détendu dépasse de 20% le diamètre d'ouverture (**Dgesp**.) des branches trachéales (**12**) se trouvent en état tendu au moment du positionnement dans la trachée.

10. Le support de trachéostome selon les revendications 1 et 8 est **caractérisé en ce que** les épaisseurs des branches trachiales (**12**) et de la branche de trachéostome (**11**) sont presque égales et dimensionnées de 1,5mm.

11. Le support de trachéostome selon la revendication 1 est **caractérisé en ce que** le cône d'étanchéité (**13**) est pourvu d'un fil (**132**) doté d'un bouchon (**133**).

12. Le support de trachéostome selon la revendication 10 est **caractérisé en ce que** le cône d'étanchéité (**13**), le fil (**132**) et le bouchon (**133**) sont fabriqués dans une seule pièce moulée par injection.

13. Le support de trachéostome selon la revendication 1 est **caractérisé en ce que** la branche de trachéostome (**11**) et les branches trachéales (**12**) sont fabriquées dans une seule pièce moulée par injection.

14. Le support de trachéostome selon la revendication 1 est **caractérisé en ce que** le dispositif de sécurité est fabriqué en forme de bague de sécurité (**14**) appliquée par injection et séparable, dont le diamètre extérieur est dimensionné de manière de pouvoir empêcher tout enlèvement accidentel du cône d'étanchéité (**13**).

15. Le support de trachéostome selon les revendications 13 et 14 est **caractérisé en ce que** la branche de trachéostome (**11**), les branches trachéales (**12**) et le cône d'étanchéité (**13**) sont fabriqués dans une seule pièce moulée par injection.

16. Le support de trachéostome selon une des revendications précédentes est **caractérisé en ce que** tous les composants support de trachéostome (**1**) sont fabriqués dans un matériau à base de silicone.
